# EUROPÄISCHE PATENTSCHRIFT

(11) **EP 2 671 609 B1**
(45) Veröffentlichungstag und Bekanntmachung des Hinweises auf die Patenterteilung: **16.09.2015**
(21) Anmeldenummer: 13002605.7
(22) Anmeldetag: 17.05.2013
(51) Int. Cl.: A61M 37/00

(54) **Tiefengenaue Kapillarnadel zum repetierenden Einbringen flüssiger Stoffe in die Haut**
Precise depth capillary needle for repeated introduction of fluids into the skin
Aiguille de capillaire à profondeur précise pour l'introduction répétée de substances liquides dans la peau

(30) Priorität: 21.05.2012 DE 102012009848
(43) Veröffentlichungstag der Anmeldung: 11.12.2013
(73) Patentinhaber: Blank, Anton, 70173 Stuttgart (DE)
(72) Erfinder: Blank, Anton, 70173 Stuttgart (DE)

(56) Entgegenhaltungen:
- EP-A1- 1 882 491
- DE-A1- 4 331 442
- US-A- 2 588 623

## Beschreibung

Tiefengenaue Kapillarnadel zum repetierenden Einbringen flüssiger Stoffe in die Haut.

### Stand der Technik

Geräte zum Einbringen von Stoffen in die Haut werden zum Beispiel zum Einbringen von in Flüssigkeiten gelösten Farbpigmenten für Permanent Make-up (PMU) oder einer Tätowierung sowie anderer kosmetischer und medizinischer Stoffe genutzt und bestehen üblicherweise aus einer Antriebseinheit (Handgerät) zur Erzeugung einer repetierenden Hubbewegung mit einer ausgefahrenen und einer eingefahrenen Stellung, die auf eine Vorrichtung, die ein Stechelement, welches mit dem einzubringenden Stoff benetzt ist, enthält, um die Haut bei jeder Stechbewegung punktuell anzustechen, damit der Stoff in die Haut eindringen und in ihr verbleiben kann.

Eine Fülle von Patentdokumenten zeigen solche Vorrichtungen. Bei allen diesen bekannten Vorrichtungen oszilliert die Nadel in einer feststehenden Hülse oder Düse, welche die einzubringende Flüssigkeit enthält, so dass die Nadel, nachdem sie in ihrer ausgefahrenen Stellung durch Einstechen in die Haut einen Teil ihrer Flüssigkeitsbenetzung zuruck gelassen hat, beim Zurückfahren in ihre eingefahrene Stellung in der Hülse oder Düse erneut mit der Flüssigkeit benetzt wird. Um die Nadelspitze vollständig mit Flüssigkeit zu benetzen bedarf es eines im Vergleich zur Nutzeinstechtiefe von ungefähr 0,1mm bis 0,3mm beispielsweise für PMU, vergleichsweise großen Nadelhubs, der in der Praxis im Bereich von ungefähr 1,5 bis über 4mm liegt, um eine ausreichende Nadelbenetzung und damit einen ausreichenden Flüssigkeitseintrag in die Haut zu gewährleisten.

Dieser vergleichsweise große Nadelhub zur ausreichenden Flüssigkeitsbenetzung der Nadel bedingt unnötig starke Antriebe mit einem in Bezug auf den Nutzhub geringen Wirkungsgrad und entsprechen unnötig großer Verlustleistung. Der größte Nachteil sind jedoch die mit der Antriebsleistung quadratisch ansteigenden Gerätvibrationen, deren Maximalamplitude, gemessen an der Nadelspitze, regelmäßig die maximale Nutzamplitude beispielsweise für PMU von ca. 0,3mm übersteigt.

Ein weiterer Nachteil der bekannten Vorrichtungen besteht in der unbekannten Stechtiefe, die das Einbringen der Flüssigkeit in zu große oder zu geringe Hauttiefen bedingt, als medizinisch und kosmetisch optimal wäre und nahezu immer ein Nacharbeiten nach dem Abheilen der Erstbehandlung mit erneuter Narbenbildung erzwingt. Die einzige Ausnahme ist in den Patentanmeldungen DE102008031907, DE102011014196 und EP12001723 vom gleichen Erfinder beschrieben, in denen der Nadelhub anhand einer Nullpunktreferenz mit einem Sensor gemessen und mit einer Regeleinrichtung auf einstellbare Sollwerte geregelt wird. Vorliegende Patentanmeldung beschreibt eine weitere Vorrichtung, um eine Flüssigkeit tiefengenau in eine Haut einzubringen.

### Die Erfindung

Aufgabe der Erfindung ist es, eine Vorrichtung zu schaffen, die mit kleinstmöglichem Nadelhub eine optimale Nadelbenetzung und somit bestmöglichen Flüssigkeitseintrag und gleichzeitig ein tiefengenaues Einbringen der Flüssigkeit in die Haut ermöglicht.

Diese Aufgabe wird erfindungsgemäß mit einer Vorrichtung nach dem Oberbegriff des unabhängigen Anspruchs 1 dadurch gelöst, dass eine Nadel mit einer Hülse fest gekoppelt ist, die so gebildete Nadel-Hülsenkombination als Ganzes durch den Antrieb in Oszillation versetzt wird, die Flüssigkeit durch eine oder mehrere Eintrittsöffnungen in der Hülse durch Kapillarwirkung in den Zwischenraum zwischen Hülseninneren und Nadel eindringt und die maximale Eindringtiefe der Nadel in die Haut durch einen Nadelüberstand über das Ende der Hülse hinaus beliebig herstellbare Eindringtiefen gestattet.

Ein wesentlicher Vorteil, welcher mit der Erfindung gegenüber dem Stand der Technik erreicht wird, besteht darin, dass der Zwischenraum zwischen Kapillarinnerem und Nadel durch Kapillarwirkung stets mit Flüssigkeit gefüllt ist und somit die ungefähr 0,1 mm bis 0,3mm über die Hülse herausstehende Nadelspitze permanent in einen Flüssigkeitsfilm sticht, der sich auf der Haut durch das periodische Aufsetzen des Endes der Nadel-Hülsenkombination bildet. Damit ist ein optimaler Flüssigkeitseintrag durch stetige Benetzung der Nadelspitze erreicht.

Ein weiterer wesentlicher Vorteil, welcher gegenüber dem Stand der Technik erreicht wird, besteht darin, dass die Hülse in dieser Vorrichtung als 'Anschlag' wirkt, so dass bei jedem Stich die exakt gleiche Tiefe gestochen wird, wie bei der Nadelfertigung vorgegeben. Durch Bereitstellung mehrerer Nadel-Hülsenkombinationen mit unterschiedlich weit herausstehender Nadelspitze, können für jeden Anwendungsfall die optimalen Eindringtiefen vorgegeben werden. In der Praxis wählt beispielsweise die Kosmetikerin die Einbringtiefe anhand der Behandlungsregion einfach durch Wechseln der Nadel aus

Ein weiterer wesentlicher Vorteil der Erfindung gegenüber dem Stand der Technik besteht darin, dass nur noch ein maximaler Nadelhub von geringfügig mehr als dem maximalen Nutzhub beispielsweise für PMU von 0,3mm erforderlich ist, dadurch die Antriebsleistung drastisch gesenkt werden kann und die damit quadratisch abnehmenden Vibrationen des Gerätes ein bedeutend präziseres Arbeiten ermöglichen.

Eine bevorzugte Möglichkeit der Ausgestaltung der Erfindung zum tiefengenauen kapillarunterstützten Einbringen flüssiger Stoffe in die Haut wird erfindungsgemäß dadurch gebildet, dass die Hülse und die Nadel ungefähr gleich lang sind und die daraus gebildete Nadel-Hülsenkombination direkt mit dem Antrieb gekoppelt ist.

Eine vorteilhafte Möglichkeit der Ausgestaltung der Erfindung zum tiefengenauen kapillarunterstützten Einbringen flüssiger Stoffe in die Haut wird erfindungsgemäß dadurch gebildet, dass die Nadel länger ist als die Hülse und die daraus gebildete Nadel-Hülsenkombination nur mit der Nadel mit dem Antrieb gekoppelt ist.

Eine zweckmäßige Möglichkeit der Ausgestaltung der Erfindung zum tiefengenauen kapillarunterstützten Einbringen flüssiger Stoffe in die Haut wird erfindungsgemäß dadurch gebildet, dass die Nadel kürzer ist als die Hülse und die daraus gebildete Nadel-Hülsenkombination nur mit der Hülse mit dem Antrieb gekoppelt ist.

Eine zweckmäßige Ausgestaltung der Erfindung sieht vor, dass ein Quetschring zur unverrückbaren Verbindung von Hülse und Nadel verwendet wird, der gleichzeitig ein Herausrutschen der Hülsen-Nadelkombination aus dem Gehäuse verhindert, beim Einsetzen der Nadel den erforderlichen Anschlagwiderstand für beispielsweise ein Klemmvorrichtung im Antrieb bereit stellt und beim Herausziehen der Hülsen-Nadelkombination einen Verbleib derselben im Antrieb verhindert.

Eine mögliche Ausgestaltungsform der Erfindung zum tiefengenauen kapillarunterstützten Einbringen flüssiger Stoffe in die Haut wird erfindungsgemäß dadurch gebildet, dass die Eintrittsöffnung oder die Eintrittsöffnungen für die Flüssigkeit in der Nadel-Hülsenkombination in ihrer Lage, in ihrer Größe und in ihrer Anzahl auf die Anwendungsflüssigkeit abgestimmt werden kann.

Eine Ausgestaltungsform der Erfindung sieht vor, dass die lichte Weite zwischen Kapillarinnerem und Nadel je nach maximaler Partikelgröße der in der Flüssigkeit befindlichen Wirkstoffe angepasst werden kann.

Eine mögliche Ausgestaltungsform der Erfindung sieht vor, dass die Nadel der Nadel-Hülsenkombination entfällt und mit der verbleibenden Hülse alleine durch Kapillarwirkung ein Flüssigkeitsfilm mit definierbarer Breite auf die Haut aufgetragen werden kann. Diese Anwendung ist beispielsweise beim PMU und beim Tätowieren zum Vorzeichnen der herzustellenden Linien und Flächen von Vorteil, da direkt mit der auch für das PMU oder der Tätowierung verwendeten flüssigen Farbe vorgezeichnet werden kann.

Erfindungsgemäß wird der Wirkungsgrad des Gesamtsystems in allen denkbaren Ausführungsformen wesentlich verbessert, so dass die erforderliche Antriebsenergie reduziert und damit die Geräteerwärmung und insbesondere die Gerätevibrationen verringert werden

Erfindungsgemäß wird tiefengenaues Einbringen von Flüssigkeiten in eine Haut unter allen Bedingungen eingehalten, solange der Anwender die Nadelspitze so fuhrt, dass das Hülsenende bei jedem Hub auf die Haut auftrifft

Die Vorrichtung zum tiefengenauen kapillarunterstützten Einbringen flüssiger Stoffe in die Haut entfaltet die beschriebenen Vorteile beim Anbringen von Permanent Make-Up, Tätowierungen auf der Haut und beim Einbringen kosmetischer und medizinischer Stoffe in definierte Tiefen in die Haut.

### Bevorzugtes Ausführungsbeispiel der Erfindung

Die Erfindung wird nachfolgend anhand eines Ausführungsbeispiels unter Bezugnahme auf eine Zeichnung näher erläutert.,

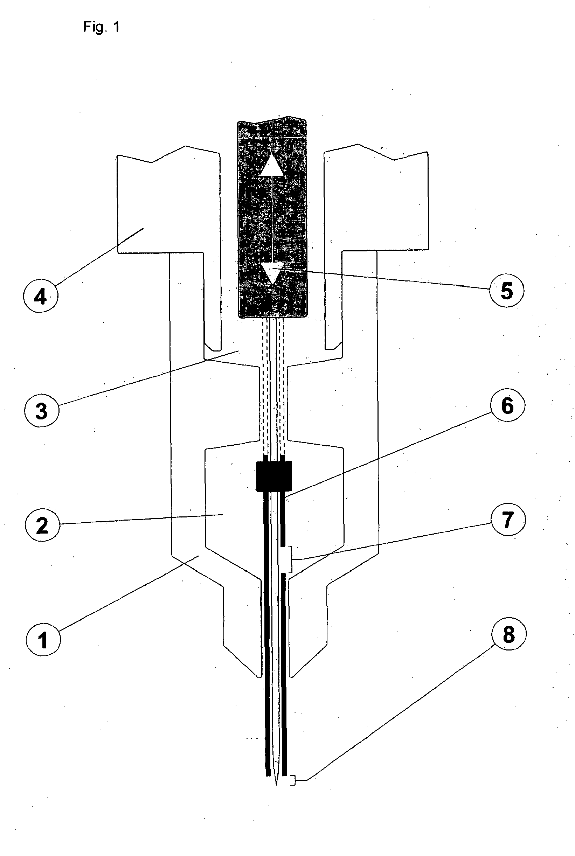

Fig. 1 zeigt eine Vorrichtung zum tiefengenauen kapillarunterstützten Einbringen flüssiger Stoffe in die Haut für Permanent-Make-up (PMU) oder einer Tätowierung oder anderer kosmetischer und medizinischer Stoffe, mit einem Gehäuse 1, das in eine vordere Flüssigkeitskammer 2 und eine hintere Antriebskammer 3 aufgeteilt ist. Das Gehäuse 1 koppelt zusammen mit der Nadel-Hülsenkombination 6 an das Handgerät 4 respektive an den Antrieb 5 der periodisch oszillierende Hübe ausführt. Durch die Einlassöffnung 7 in der Nadel-Hülsenkombination dringt die in der Flüssigkeitskammer 2 befindliche Flüssigkeit durch Kapillarwirkung und unterstützt durch die oszillierende Bewegung der Nadel-Hülsenkombination rasch in den Zwischenraum zwischen Kapillarinnerem und Nadel ein und erreicht nach kurzer Zeit, im Millisekundenbereich, das untere Hülsenende mit der aus dieser herausragenden Nadelspitze 8.

Fährt der Antrieb die Nadel-Hülsenkombination in ihre ausgefahrene Stellung, der während des Ausfahrens auf eine Haut trifft, so sticht die Nadel einen Teil der Flüssigkeit so tief in die Haut ein, wie die Nadelspitze aus der Hülse herausragt. Durch die Elastizität der Haut dringt die wesentlich dickere und stumpfere Hülse niemals in die Haut ein, sondern stößt die Haut mit eingedrungener Nadelspitze vor sich her. Wird vom unteren Hülsenende Flüssigkeit abgezogen, wird die kapillare Flüssigkeitssäule aus der Farbkammer aufgefüllt. Dies geschieht bei jedem Hub und kontinuierlich, da die Kapillarkräfte spaltabhängig bei Weitem groß genug sind, um die Flüssigkeitssäule nicht abreißen zu lassen.

Die in der vorstehenden Beschreibung, der Zeichnung und den Ansprüchen offenbarten Merkmale der Erfindung können sowohl einzeln als auch in beliebiger Kombination für die Verwirklichung der Erfindung in ihren verschiedenen Ausführungsformen von Bedeutung sein. Der Schutzgegenstand wird durch die zugehörigen Ansprüche definiert

## Patentansprüche

1. Vorrichtung zum tiefengenauen kapillarunterstützten repetierenden Einbringen flüssiger Stoffe in die Haut, insbesondere für Permanent-Make-up (PMU) oder einer Tätowierung, mit:
- einem Gehäuse 1,
- einer im Gehäuse 1 geführten, axial verschiebbaren Nadel-Hülsenkombination 6,
- einer Flüssigkeitskammer 2 und einer Antriebskammer 3, die in dem Gehäuse 1 gebildet sind,
**dadurch gekennzeichnet, dass** mit Hilfe eines Antriebs 5 die Nadel-Hülsenkombination 6 in eine repetierende Axialbewegung versetzt wird.

2. Vorrichtung nach Anspruch 1, **dadurch gekennzeichnet, dass** die Hülse und die Nadel der Nadel-Hülsenkombination 6 unverrückbar miteinander gekoppelt sind.

3. Vorrichtung nach Anspruch 2, **dadurch gekennzeichnet, dass** die Nadel-Hülsenkombination 6 eine oder mehrere Eintrittsöffnungen 7 für die in der Flüssigkeitskammer 2 befindliche Flüssigkeit besitzt.

4. Vorrichtung nach den Ansprüchen 1 bis 3, **dadurch gekennzeichnet, dass** zur Festlegung der maximalen Eindringtiefe der Nadelspitze 8, diese um das gewünschte Maß aus der Hülse der Nadel-Hülsenkombination 6 herausragt, während das Hülsenende als Anschlag wirkt.

5. Vorrichtung nach Anspruch 3, **dadurch gekennzeichnet, dass** die Kapillarwirkung durch die mindestens eine Eintrittsöffnung 7 nur zum distalen Ende der Nadel-Hülsenkombination 6 wirkt, indem das zum Antrieb zeigende Ende der Hülse luftdicht verschlossen ist..

6. Vorrichtung nach Anspruch 1 bis 5, **dadurch gekennzeichnet, dass** die unverrückbare Verbindung zwischen Hülse und Nadel durch eine Quetschhülse erfolgt, die gleichzeitig ein Herausrutschen der Nadel in beiden Richtungen verhindert.

7. Vorrichtung nach einem der vorangegangenen Ansprüche, **dadurch gekennzeichnet, dass** die lichte Weite zwischen Kapillarinnerem und Nadel der Nadel-Hülsenkombination 6 an die maximale Partikelgröße der zu verarbeitenden Flüssigkeit angepasst wird.

8. Vorrichtung nach einem der vorangegangenen Ansprüche, **dadurch gekennzeichnet, dass** das Gehäuse 1 zusammen mit der Nadel-Hülsenkombination 6 als ein Einwegelement ausgeführt ist, das lösbar an einer Antriebseinheit 4 koppelbar ist.

9. Verwendung einer Vorrichtung nach einem der vorangegangenen Ansprüche zum Anbringen von Permanent-Make-up (PMU) oder einer Tätowierung in die Haut.

## Claims

1. An apparatus for capillary-aided precise depths repetitive application of liquid substances into the skin especially for Permanent-Make-up (PMU) or a Tattoo, comprising:
- a housing 1
- guided and axial movable needle-sleeve combination 6 in the housing 1
- a liquid-chamber 2
- and a drive-chamber 3 which are comprised in the housing 1
wherein the needle-sleeve combination will be forced into a repetitive axial movement by the aid of a drive 5.

2. The apparatus as in claim 3, wherein the sleeve and the needle of the needle-sleeve combination 6 are rigidly connected with each other.

3. The apparatus as in claim 3, wherein the needle-sleeve combination 6 has one or more inlet orifices 7 for the liquid in the liquid-chamber 2.

4. The apparatus according to claim 1 to 3, wherein for fixing the penetration depth of the needle point 8 the distal end of the needle protrudes from the sleeve of the needle-sleeve combination by the desired measure while the end of the sleeve acts as mechanical stop.

5. The apparatus as in claim 3, wherein the capillary action through the minimum of one inlet orifice 7 only works to the distal end of the needle-sleeve combination as the end of the sleeve showing to the drive is closed in an airtight way.

6. The apparatus according to claim 1 to 5, wherein the rigid connection between sleeve and needle is performed by means of a ferrule which concurrently prevents a slip out of the needle in both directions.

7. The apparatus as in claim 1, wherein the inner width of the capillary and the needle of the needle-sleeve combination 6 will be adapted to the maximal particle-size of the liquid to be used.

8. The apparatus according to one of the preceding claims, wherein the housing 1 together with the needle-sleeve combination 6 is carried out as a one-way element which is coupleable and detachable to a drive 4.

9. Use of an apparatus according to one of the preceding claims for applying Permanent Make-Up (PMU) or Tattoos into the skin.

## Revendications

1. Dispositif d'apport précis et répété par capillarité de substances liquides dans la peau, notamment utilisé pour le permanent make-up (PMU ou maquillage permanent) ou un tatouage, doté
- d'un boîtier 1
- d'une combinaison 6 associant une aiguille et un manche ajustables sur un axe
- d'une chambre de rétention de liquide 2
- et d'une chambre d'entraînement 3 montées dans le boîtier 1
**caractérisé par le fait que** la combinaison aiguille - manche 6 effectue un mouvement répété par rapport à un axe grâce à un moteur 5.

2. Dispositif conforme à l'exigence 1 **caractérisé par le fait que** le manche et l'aiguille de la combinaison aiguille - manche 6 sont immuablement accrochés ensembles.

3. Dispositif conforme à l'exigence 2 **caractérisé par le fait que** la combinaison aiguille - manche 6 dispose d'une ou plusieurs ouvertures d'entrée 7 pour le liquide qui se trouve dans la chambre de rétention de liquide 2.

4. Dispositif conforme aux exigences 1 à 3 **caractérisé par le fait que** la pointe de l'aiguille 8 sort du manche de la combinaison aiguille - manche 6 d'une longueur souhaitée afin de définir la profondeur de pénétration maximale tandis que l'extrémité du manche agit comme une butée.

5. Dispositif conforme à l'exigence 3 **caractérisé par le fait que** l'effet de capillarité provoqué par une ouverture d'entrée 7 au moins agit seulement à l'extrémité distale de la combinaison 7aiguille-manche 6 en fermant hermétiquement l'extrémité de manche dirigée vers le moteur.

6. Dispositif conforme aux exigences 1 à 5 **caractérisé par le fait que** la liaison fixe entre le manche et l'aiguille s'effectue via une douille de pincement qui dans le même temps empêche l'aiguille de glisser des deux côtés.

7. Dispositif conforme à l'une des exigences précédentes **caractérisé par le fait que** la largeur intérieure entre le capillaire et l'aiguille de la combinaison aiguille - manche 6 s'adapte à la taille maximale des particules du liquide avec lequel on travaille.

8. Dispositif conforme à l'une des exigences précédentes **caractérisé par le fait que** le boîtier 1 forme un élément jetable avec la combinaison aiguille - manche 6, élément détachable que l'on accroche à l'unité d'entraînement 4.

9. Utilisation d'un dispositif conforme à l'une des exigences précédentes destiné à l'apport de permanent make-up (PMU) ou d'un tatouage dans la peau.
